# EUROPEAN PATENT SPECIFICATION

(11) **EP 3 878 519 B1**
(45) Date of publication and mention of the grant of the patent: **14.08.2024**
(21) Application number: 21160615.7
(22) Date of filing: 04.03.2021
(51) Int. Cl.: A61Q 17/04, A61K 8/37, A61K 8/41, A61K 8/49

(54) **TOPICAL SUN PROTECTION COMPOSITION**
TOPISCHE SONNENSCHUTZZUSAMMENSETZUNG
COMPOSITION TOPIQUE DE PROTECTION SOLAIRE

(30) Priority: 09.03.2020 LU 101670
(43) Date of publication of application: 15.09.2021
(73) Proprietor: Société De Recherche Cosmétique S.à.r.L., 2314 Luxembourg (LU)
(72) Inventor: NOUAL, Amandine, 1017 VG Amsterdam (NL)
(74) Representative: Lecomte & Partners

(56) References cited:
- EP-A1- 3 308 836
- EP-A1- 3 351 236
- US-A1- 2007 185 057
- US-A1- 2015 202 131
- US-A1- 2015 209 259
- US-A1- 2018 015 022
- US-A1- 2020 030 197
- US-B1- 6 409 997
- "Suncare compositions with new cosmetic raw materials (4) ED - Darl Kuhn", IP.COM, IP.COM INC., WEST HENRIETTA, NY, US, 18 August 2011 (2011-08-18), XP013147058, ISSN: 1533-0001

## Description

### Technical field

The invention is directed to the field of cosmetics, and more particularly to sun protection topical compositions with a high Sun Protection Factor (SPF).

### Background art

It is well known that ultraviolet (UV) radiations, in particular UVA and UVB, are harmful to human skin and cause different types of skin damages. Indeed, UVB radiations (wavelength between 290 to about 320 nm) are responsible for sunburns and account for some skin cancers. Upon these radiations, epidermal biomechanical properties undergo long term detrimental changes, leading to premature ageing of the skin. For a long time considered as less harmful than UVB, it has been shown that UVA radiations (wavelength between 320 nm and 400 nm) while producing tanning of the skin, contribute also to sunburns. Moreover, these UVA radiations, under quite normal everyday conditions, are also sufficient to damage over time the collagen and elastin fibres, which are important elements for the structure and strength of the skin.

Therefore, to protect the human skin against sunlight and particularly against UVA and UVB radiations, it is thus desirable that the sun protection products, which are applied directly on the skin, provide UV filtering over the range of UVA and UVB.

A range of UV filters against UVA, UVB or both which can be used in cosmetic sun protection compositions have been developed and are available for the cosmetic industry. In most countries, these UV filters are in particular summarized in the form of approved lists, such as Annex VI of the European Cosmetic Regulation n°1223/2009 which authorizes 30 UV filters.

Typically, two types of UV filters enable skin photo-protection against UVA and/or UVB radiations, namely organic filters and mineral filters. Organic filters, also called chemical filters, absorb UV radiations instead of the skin. Some of these organic filters are liquid and the others are under a solid form (i.e. in powder form) but they are mainly liposoluble. In contrast, inorganic UV filters which are also called physical filters, reflect the UV radiations, and they are inert and opaque powders.

Commonly, the protection of sun products against UVB radiations is reflected by the sun protection factor (SPF). This factor indicates the protection that the human skin gets from exposure to these radiations, and it is the foremost criteria watched and demanded by the consumers, in parallel with getting sunscreen products which are not heavy, oily, and/or give a sticky sensation on the skin.

One of the ways of achieving a high protection against sun's UV, is to incorporate high levels of UVA, UVB and broad spectrum filters in the sun protection products. Organic filters achieve protection mainly by absorbing the UV radiations whereas inorganic filters mainly reflect the UV radiations. Under certain circumstances, inorganic filters often involving nanoparticles might not be desirable for the consumers and the environment. However, effective known organic UV filters, particularly solid lipophilic organic UV filters, when they are used in high concentration to prepare sunscreen products, present a problem of solubility and moreover lead to unstable sunscreen products since these filters tend to (re)crystallise over time. This can occur in different galenic forms of the sun protection products/compositions. A consequence of this (re)crystallisation is that the UV protection of the sunscreen products is significantly decreased.

Moreover, lipophilic organic UV filters, when they are used in a sun topical protection product, conduct to sun products presenting a heavy, oily and sticky sensation leading to a discomfort when applied on the skin. In the case of solid sun protection compositions with a high level of lipophilic solid organic UV filters, these undesired sensorial properties are moreover increased by an unattractive visual appearance. Problems of solubility and (re)crystallisation of high concentration of solid lipophilic organic UV filters can provide solid sun protection compositions having a rough surface; i.e. not enough smooth and/or presenting cracks. This actually tends to decrease the efficiency of the spreading of these compositions on the skin leading to a poor UV protection.

To solve the problems of solubility of solid organic UV filters, and more particularly lipophilic organic filters, the liquid organic UVB filter ethylhexyl-2-cyano-3,3-diphenylacrylate (INCI: octocrylene) has been used for a long time by cosmetic formulators as emollient ester/solvent and also as photo-stabilizer. Similarly, the (lipophilic) organic UV filter, ethyhexyl methoxycinnamate, is also widely used to dissolve and stabilize solid lipophilic organic UVA and UVB filters, respectively, in addition to its function of UV absorber. However, these two UV filters are controversial for being suspected to have a negative impact on human health and the environment.

Concerning the undesired sensorial properties of sun topical protection products containing lipophilic organic UV filters, this problem is most commonly solved by the addition of siloxanes and or derivatives, such as dimethicone, which are well known in the cosmetic industry for their high sensorial properties. They are particularly used to reduce the sticky and greasy feeling on the skin. However, siloxanes and derivatives are currently being avoided due to their ecological footprint and their potential occlusive effect on the skin.

### Summary of the invention

### Technical Problem

The invention has for technical problem to overcome at least one of the drawbacks of the prior art. More specifically, the invention has for technical problem to provide stable sun topical protection compositions over time with a high SPF, preferably associated with a transparent appearance and a light sensation on the skin, a non-greasy sensory effect and an easy and/or pleasant spreadability.

### Technical solution

Surprisingly, the objects of this invention are achieved by a sun protection topical composition comprising a UV filter system achieving a Sun Protection Factor SPF of at least 30; at least two ester emollients comprising dibutyl adipate with a mass that is at least 89.5% of the total mass of the UV filter system and butyloctyl salicylate with a mass that is at least 24% of the total mass of the UV filter system.

Advantageously, the mass of dibutyl adipate may be at least 95% of the total mass of the UV filter system.

According to a preferred embodiment, the UV filter system may be a powder based by at least 60%, preferably at least 70%, more preferably 100%, in mass.

According to a preferred embodiment, the UV filter system may comprise at least three different UV filters, preferably lipophilic and organic UV filters.

According to a preferred embodiment, each one of the different filters may present a mass that is not less than 33% and/or not more than 300% of the mass of each other one of said different filters.

According to a preferred embodiment, the UV filter system may comprise filters selected from the group consisting of ethylhexyl triazone, diethylamino hydroxybenzoyl hexyl benzoate, bis-ethylhexyloxyphenol methoxyphenyl triazine, ethylhexyl salicylate, diethylhexyl butamido triazone and homosalate.

According to a preferred embodiment, the UV filter system may comprise at least one UVA filter and at least one UVB filter, and/or at least one UVA+UVB (broad spectrum) filter.

According to a preferred embodiment, the at least two ester emollients may present a total mass of ester emollients that is comprised between 1.2 and 4.3 times the total mass of the UV filter system.

According to a preferred embodiment, the at least two ester emollients may further comprise at least one compound selected from the group consisting of triheptanoin, with a mass that is at least 51% of the total mass of the UV filter system, hexyl laurate, with a mass that is at least 32% of the total mass of the UV filter system, isoamyl laurate, with a mass that is at least 65% of the total mass of the UV filter system, and diisopropyl sebacate, with a mass that is at least 40.5%, preferably at least 49%, of the total mass of the UV filter system, or mixtures thereof.

According to a preferred embodiment, the at least two ester emollients may present a total mass concentration that is comprised between 23.6 and 80%, or preferably between 23.6 and 75%, or preferably between 41.3 and 80% based on the total sun protection composition.

In some alternate embodiments, the at least two ester emollients may present a total mass concentration that is comprised between 23.6 and 60%, preferably between 23.6 and 40%, based on the total sun protection composition.

The expression "based on the total sun protection composition" means the % is defined according to 100 wt% of the sun protection composition.

According to a preferred embodiment, the sun protection topical composition may further comprise at least one oil thickener; so that said sun protection topical composition is solid at room temperature for forming a stick.

According to a preferred embodiment, the at least one oil thickener may comprise amino acid based oil gelling agents.

According to a preferred embodiment, the at least one oil thickener may present a total mass concentration that is comprised between 15% and 25% based on the total sun protection composition, when the UV filter system is of from 23.6 to 40% based on the total sun protection composition.

In some alternate embodiments, the at least one oil thickener may be present in the sun protection composition at a total mass concentration that is comprised between 5% and 20% based on the total sun protection composition.

According to a preferred embodiment, the sun protection topical composition may further comprise at least one film former.

According to a preferred embodiment, the at least one film former may present a total mass concentration that is comprised between 0.5% and 3.5% with respect to the total sun protection composition.

According to a preferred embodiment, the UV filter system may present a total mass concentration that is comprised between 17 and 35%, preferably between 18 and 23% with respect to the total sun protection composition.

According to a preferred embodiment, the sun protection topical composition may be anhydrous.

According to a preferred embodiment, the sun protection topical composition may be free of at least one compound selected from the group consisting of siloxanes, elastomer, octocrylene, butyl methoxydibenzoylmethane, ethylhexyl methoxycinnamate, titanium dioxide and zinc oxide, or mixture thereof.

According to a preferred embodiment, the sun protection topical composition is free of siloxane derivative.

According to a preferred embodiment, the sun protection topical composition is free of elastomer.

According to a preferred embodiment, the sun protection topical composition is free of octocrylene, butyl methoxydibenzoylmethane, and/or ethylhexyl methoxycinnamate.

According to a preferred embodiment, the sun protection topical composition further comprising water, the sun protection topical composition being an emulsion, a gel-cream, a foam or a bi-phasic.

### Advantages of the invention

The invention is particularly interesting in that it provides sun protection topical compositions with a high SPF in which the UV filter system comprises a high level in mass of organic UV filters which are solubilized in a stable manner over time and at different temperatures. This invention is all the more interesting in that the UV filter system, which comprises at least 60%, preferably at least 70% and more preferably at least 100%, of powered based organic UV filters, is therefore prevented to crystallise or re-crystallise. This invention is also even more interesting in that it provides sun protection topical compositions characterized by a high SPF, a transparent appearance and a light, non-greasy texture upon application on the skin. Even more interestingly, this invention has for advantage to provide more particularly anhydrous and stable sun protection topical compositions with a high SPF in stick, gel and oils forms, these compositions being free of siloxane derivative but also free of octocrylene, butyl methoxydibenzoylmethane and ethylhexyl methoxycinnamate, and as well as of titanium dioxide and zinc oxide. Concerning the solid forms in stick, these compositions present a good balance between hardness, transparency and light sensory properties, and an easy and pleasant spreadability upon application. Another advantage of this invention is that it may be possible to use the combination of the UV filter system and the at least two esters emollients, as an oil phase to make stable galenic forms containing water (i.e. emulsions, gel creams, foams and bi-phases).

### Detailed description of the invention

The present invention provides a base for a sun protection topical composition which comprises a UV filter system with a Sun Protection Factor of at least 30, preferably 50 and above, and emollients. The emollients comprise at least two esters emollients being dibutyl adipate and butyloctyl salicylate. It has been found out that certain concentrations of these emollients in combination with the filter system provide good results with regard to solubilisation of the powder-based filters and also advantageous characteristics for topical applications like transparency and sensorial properties.

The two ester emollients can be present respectively in the sun protection topical composition with a mass that is at least 87% and 24% of the total mass of the UV filter system. Moreover, this composition is free of siloxane derivative.

The at least two ester emollients can comprise triheptanoin with a mass that is at least 51 % of the total mass of the UV filter system.

The at least two ester emollients can also further comprise hexyl laurate with a mass that is at least 32% of the total mass of the UV filter system.

The at least two ester emollients can further comprise isoamyl laurate with a mass that is at least 65% of a total mass of the UV filter system and/or diisopropyl sebacate with a mass that is at least 40.5% of the total mass of the UV filter system.

The UV filter system of the sun protection topical composition is formed with organic UV filters. This UV filter system may be a powder based by at least 60%, preferably at least 70%, more preferably 100% in mass. By powder based, it is understood that the UV filters are under solid form.

The UV filter system can comprise at least three different lipophilic organic UV filters. The at least three different lipophilic organic UV filters can comprise: diethylamino hydroxybenzoyl hexyl benzoate, bis-ethylhexylloxyphenol methoxyphenyl triazine and/or ethylhexyl triazone. The UV filter system can comprise only these three filters. Alternatively, the UV filter system can comprise one or more additional filters.

The UV filter system can show a balanced composition, meaning that each specific filter is at a concentration that is not too different from the concentrations of the other filters. Each one of the different UV filters can show a mass that is not more than 300%, preferably 200% and not less than 33%, preferably 50%, of the mass of each other one of said different filters.

The organic filters of the UV filter system can be selected from the group consisting of diethylamino hydroxybenzoyl hexyl benzoate, bis-ethylhexylloxyphenol methoxyphenyl triazine, ethylhexyl triazone, ethylhexyl salicylate, diethyl butamido triazone, and homosalate.

The UV filter system comprises at least one UVA, and at least one UVB, and/or at least one broad spectrum UV filter.

The above describes a base for a sun protection topical composition which can be used in various forms, i.e. as an oil, a gel, a stick (solid), an emulsion, or as a biphase.

The following examples will more fully illustrate the preferred embodiments within the scope of the invention. These examples are however intended to illustrate the present invention without thereby limiting it. Percentages indicated in the tables are based on the mass and the total amount of the sun protection topical composition provided.

### EXAMPLE 1:

This example shows the composition (Table 1) and the method of manufacturing of sun protection topical oils according to the invention. The sunscreen oils of this example 1 further contain fragrance and active ingredient. These oils present a SPF of 41 with a good stability over time at 4°C, 25°C and 40°C.

In section B), the SPF data of the sun protection oils have been determined *in silico* with the BASF sunscreen simulator (https://www.sunscreensimulator.basf.com).

In section C), the evaluation of the UV filters solubility in the ester emollients system (oil phase) and in the entire formulation (entire oil) at the different above temperatures has been performed by visual and under polarized light microscopy (X10 polarized) observations to determine the presence or not of crystals of organic UV filters. No crystallisation or re-crystallisation has been observed.

In section D), the sun protection topical oils present a high transparency and are easy to spread and not sticky. The sensorial properties of the oils have been evaluated by an internal panel. Eighty µl of each oil have been evaluated by applying on the forearms of the panellists (20 turns). The scale of measurements is: +++: high; ++: medium; +: low.

**Table I**

| **A)** | INCI name | Ex.1 (% w/w) | Ex.2 (% w/w) |
|---|---|---|---|
| | Diethylamino hydroxybenzoyl hexyl benzoate | 9.0 | 9.0 |
| | Ethyhexyl triazone | 4.5 | 4.5 |
| | Bis-ethylhexyloxyphenol methoxyphenyl triazine | 5.0 | 5.0 |
| | Hexyl laurate | 9.0 | - |
| | Dibutyl adipate | 30.0 | 34.0 |
| | Triheptanoin | 35.0 | 40.0 |
| | Butyloctyl salicylate | 6.0 | 6.0 |
| | Active ingredient | 0.5 | 0.5 |
| | Fragrance | qs | qs |
| | Total | 100.0 | 100.0 |
| | | | |
| **B)** | SPF (*in silico*) | 41 | 41 |
| | | | |
| **C)** | Stability of the oil phase (6 months at 4°C, 25°C, and 3 months at 40°C) | No (re)crystallisation | No (re)crystallisation |

| | Stability of the entire oil (6 months at 4°C, 25°C, and 3 months at 40°C) | No (re)crystallisation | No (re)crystallisation |
|---|---|---|---|
| | | | |
| **D)** | Transparency | +++ | +++ |
| | Spreadability | +++ | +++ |
| | Shine (film) | +++ | ++ |
| | Ease of absorption | + | +++ |
| | Stickiness | + | + |
| | Oiliness | +++ | ++ |

### Manufacturing procedure:

- Weigh the emollient esters and UV filters together and stir under moderate agitation while heating at 75°C;
- Cool down to 30°C to add the fragrance and active ingredient, under moderate agitation.

### EXAMPLE 2:

This example shows the composition (Table 2) and the method of manufacturing of sun protection topical gels according to the invention. These sunscreen gels present a SPF superior to 40, and are stable without re-crystallisation of the organic UV filters over time at 4°C, 25°C and 40°C.

In section B), the SPF data of the sun protection gels have been obtained with the BASF sunscreen simulator.

In section C), the evaluation of the UV filters solubility in the ester emollients system (oil gel) and in the entire formulation (entire gel) at the different above temperatures has been performed by visual and by under polarized light microscopy (X10 polarized) observations. No crystallisation or re-crystallisation has been observed.

In section D), the sun protection topical gels have a good transparency and are easy to spread. The sensorial properties of the gels tested have been evaluated by an internal panel. Sixty µl of each gel have been evaluated when applied on the forearms of the panellists (20 turns). The scale of measurements is: +++: high; ++: medium; +: low.

**Table 2**

| **A)** | INCI name | Ex.3 (% w/w) | Ex.4 (% w/w) |
|---|---|---|---|
| | Diethylamino hydroxybenzoyl hexyl benzoate | 9.0 | 9.0 |
| | Ethylhexyl triazone | 4.5 | 4.5 |
| | Bis-ethylhexyloxyphenol methoxyphenyl triazine | 5 | 5 |
| | Dibutyl adipate | 33.0 | 64.0 |
| | Triheptanoin | 35.0 | - |
| | Butyloctyl salicylate | 6.0 | 10.0 |
| | Film former | 3.0 | 3.0 |
| | Active ingredient | 1.0 | 1.0 |
| | Fragrance | qs | qs |
| | Preservative system | 0.5 | 0.5 |
| | Antioxidant | 0.5 | 0.5 |
| | Active ingredient | 0.5 | 0.5 |
| | Total | 100.0 | 100.0 |
| | | | |
| **B)** | SPF *in silico* | 41 | 41 |
| **C)** | Stability of the oil phase (6 months at 4°C, 25°C and 3 months at 40°C) | No (re)crystallisation | No (re)crystallisation |
| | Stability of the entire gel (6 months at 4°C, 25°C and 40°C) | No (re)crystallisation | No (re)crystallisation |
| | | | |
| **D)** | Transparency | +++ | +++ |
| | Spreadability | ++ | +++ |
| | Shine (film) | ++ | +++ |
| | Ease of absorption | +++ | + |
| | Oily | +++ | +++ |

### Manufacturing procedure:

- Add the film former in emollient esters under rotor stator while heating up to 82°C until fully homogenous;
- Add the organic UV filters and stir under normal agitation until completely homogenous while maintaining the temperature at 75°C;
- Cool down until room temperature while stirring;
- Add the active ingredient, the antioxidant and fragrance below 30°C.

### EXAMPLE 3:

This example shows the composition (Table 3) and the method of manufacturing of sun protection topical stick according to the invention. The sunscreen sticks of this example 3 further contain an oil thickener, a film former and also fragrance and active ingredient. These solid compositions in form of stick present a SPF of 41 to more 60 and a good stability over time at 4°C, 25°C and 40°C. The stability has been tested at the same time on the oil phase of the sticks and separately on the entirety of the sticks.

In section B), the SPF data of the sun protection sticks have been determined *in silico* with the BASF sunscreen simulator.

In section C), the SPF data of the sun protection sticks have been screened *in vivo* following the standard method ISO 24444.

In section D), the evaluation of the UV filters solubility in the ester emollients system (oil phase) and in the entire formulation (entire stick) at the different above temperatures has been performed by visual and under polarized light microscopy (X10 polarized) observations. No crystallisation or re-crystallisation has been observed.

In section E), these sun protection topical sticks present a good transparency, are easy to spread, and not sticky or only very slightly. The sensorial properties of the sticks have been evaluated by an internal panel. The scale of measurements is: +++: high; ++: medium; +: low.

**Table 3**

| **A)** | INCI name | Ex.5 (% w/w) | Ex.6 (%w/w) | Ex.7 (%w/w) | Ex.8 (%w/w) | Ex.9 (%w/w) | Ex. 10 (%w/w) |
|---|---|---|---|---|---|---|---|
| | Diethylamino hydroxybenzoy I hexyl benzoate | 9.0 | 9.0 | 9.0 | 9.0 | 9.0 | 9.0 |
| | Ethylhexyl triazone | 4.5 | 4.5 | 4.5 | 4.5 | 4.5 | 4.5 |
| | Bisethylhexyloxyp henol methoxyphenyl triazine | 5.0 | 5.0 | 4.5 | 5.0 | 5.0 | 5.0 |
| | Ethylhexyl salicylate | - | - | 4.5 | - | - | - |
| | Diethylhexyl butamido triazone | - | - | 3.0 | - | - | - |
| | Homosalate | - | - | 9.0 | - | - | - |
| | Hexyl laurate | - | - | - | 6.0 | - | 6.0 |
| | Dibutyl adipate | 17.5 | 17.5 | 31.3 | 25.0 | 26.0 | 25.5 |
| | Triheptanoin | 13.5 | 9.5 | - | 19.3 | 24.3 | 17.8 |
| | Butyloctyl salicylate | 4.5 | 10.0 | 10.0 | 6.0 | 6.0 | 6.0 |
| | Isoamyl laurate | 12.0 | 12.0 | - | - | - | - |
| | Diisopropyl sebacate | 9.0 | 7.5 | - | - | - | - |
| | Oil thickener | 20.0 | 20.0 | 20.0 | 20.0 | 20.0 | 20.0 |
| | Film former | 2.0 | 2.0 | 1.0 | 2.0 | 2.0 | - |
| | Fragrance | qs | qs | qs | qs | qs | qs |
| | Antioxidant | 0.5 | 0.5 | 0.5 | 0.5 | 0.5 | 0.5 |
| | Preservative system | 0.5 | 0.5 | 0.5 | 0.5 | 0.5 | 0.5 |
| | Active ingredient | 0.5 | 0.5 | 1.0 | 1.0 | 1.0 | 1.0 |
| | Water proofing film former | - | - | - | - | - | 3.0 |
| | Total | 100.0 | 100.0 | 100.0 | 100.0 | 100.0 | 100.0 |
| **B)** | SPF *in silico* | 41 | 41 | 63.5 | 41 | 41 | 41 |
| | | | | | | | |
| **C)** | SPF screening *in vivo* (Mean) | Not tested | 50.7 | 64.1 | 58.3 | 69.9 | Not tested |
| | | | | | | | |
| **D)** | Stability of the oil phase (6 months at 4°C, 25°C, and 3 months at 40°C) | No (re)cryst allisation | No (re)cryst allisation | No (re)cryst allisation | No (re)cryst allisation | No (re)cryst allisation | No (re)cryst allisation |
| | Stability of the entire stick (6 months at 4°C, 25°C, and 3 months at 40°C) | No (re)cryst allisation | No (re)cryst allisation | No (re)cryst allisation | No (re)cryst allisation | No (re)cryst allisation | No (re)cryst allisation |
| **E)** | Transparency | +++ | +++ | +++ | ++ | ++ | + |
| | Hardness | ++ | ++ | ++ | +++ | +++ | + |
| | Spreadability | +++ | + | ++ | ++ | ++ | ++ |
| | Shine (film) | + | + | ++ | +++ | + | +++ |
| | Stickiness | Slightly sticky | Slightly sticky | Not sticky | Not sticky | Not sticky | Not sticky |

### Manufacturing procedure:

- In the main vessel, weigh the esters and heat them at 85°C;
- Under emulsifier, add the film former, increase progressively the speed of the emulsifier until homogenous dispersion of the mix while keeping the temperature at 80-85°C;
- Put the homogenous dispersion under dispersion turbine and incorporate progressively organic UV filters and then reduce the agitation at the minimum until complete homogenisation while keeping the temperature at 80°C;
- Heat the oil thickener at 105°C until it becomes completely homogenous and transparent, then pour the oil thickener in the mix of esters, filters and film former;
- Decrease the temperature at 75°C to add fragrance and active ingredients;
- Stop the agitation and leave the mix to cool down;
- Few hours later, re-heat progressively the mix at 105°C and pour it in stick containers;
- Cool down sun sticks until they become hard.

### EXAMPLE 4:

This example shows the composition (Table 4) and the method of manufacturing of a sun protection topical lotion with an oil phase (UV filters and emollients) according to the invention. This sunscreen lotion presents a SPF of 41 and shows a good stability over time at 4°C, 25°C and 40°C.

In section B), the SPF data of the sun protection topical lotion of the example 4 has been determined *in silico* with the BASF sunscreen simulator.

In section C), the evaluation of UV filters solubility in the lotion at the different above temperatures has been performed by visual and under polarized light microscopy (X10 polarized) observations. No crystallisation or re-crystallisation has been observed.

**Table 4**

| **A)** | INCI name | Ex.11 (% w/w) |
|---|---|---|
| | Diethylamino hydroxybenzoyl hexyl benzoate | 9.0 |
| | Ethylhexyl triazone | 4.5 |
| | Bis-ethylhexyloxyphenol methoxyphenyl triazine | 5.0 |
| | Dibutyl adipate | 17.6 |
| | Butyloctyl salicylate | 6.0 |
| | Aqua | qsp 100 |
| | Microcrystalline cellulose gum | 1.0 |
| | Glycol(s) | 2.0 |
| | Xanthan gum | 0.1 |
| | Polyglyceryl-2 dipolyoxystearate, lauryl glucoside, glycerin, aqua, citric acid | 6.0 |
| | Potassium cetyl phosphate | 0.5 |
| | pH adjuster | qs |
| | Preservative system | 1.0 |
| | Active ingredient | 0.5 |
| | Fragrance | qs |
| | Total | 100.0 |
| **B)** | SPF *in silico* | 41 |
| | | |
| **C)** | Stability of the lotion (6 months at 4°C, 25°C, and 3 months at 40°C) | No (re)crystallisation |

### Manufacturing procedure:

- Disperse the microcrystalline cellulose & cellulose gum in the water in the main vessel. Heat up to 80°C. Premix the glycol(s), the preservative system and the xanthan gum, then add into the main vessel;
- Prepare the oil phase by mixing the esters and filters together and stir under moderate agitation while heating at 75°C;
- When both phases are hot, transfer the oil phase into the main vessel under a rotor-stator and emulsify until well homogenous;
- Cool down at room temperature while stirring under moderate agitation;
- Adjust the pH if necessary;
- Below 30°C add the fragrance and the active ingredient.

### EXAMPLE 5:

This example shows the composition (Table 5) and the method of manufacturing of sun protection topical bi-phases with an oil phase (UV filters and emollients) according to the invention. The sunscreen bi-phases of this example 5 present a SPF of 41 with a good stability over a storing at 4°C, 25°C and 40°C.

In section B), the SPF data of the sun protection bi-phases have been determined *in silico* with the BASF sunscreen simulator.

In section C), the evaluation of UV filters solubility in the oil phase of the bi-phases at the different above temperatures has been performed by visual and under polarized light microscopy (X10 polarized) observations. The ratio water phase:oil phase in the example 5 is 40:60 and 30:70.

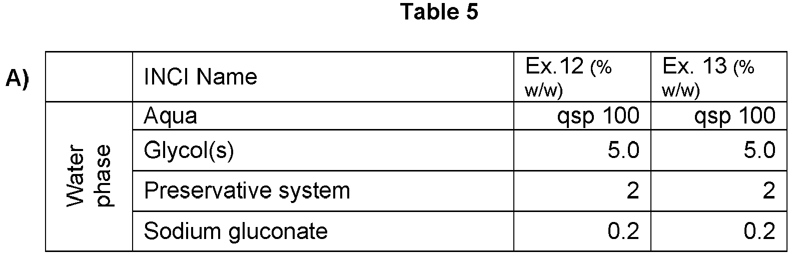

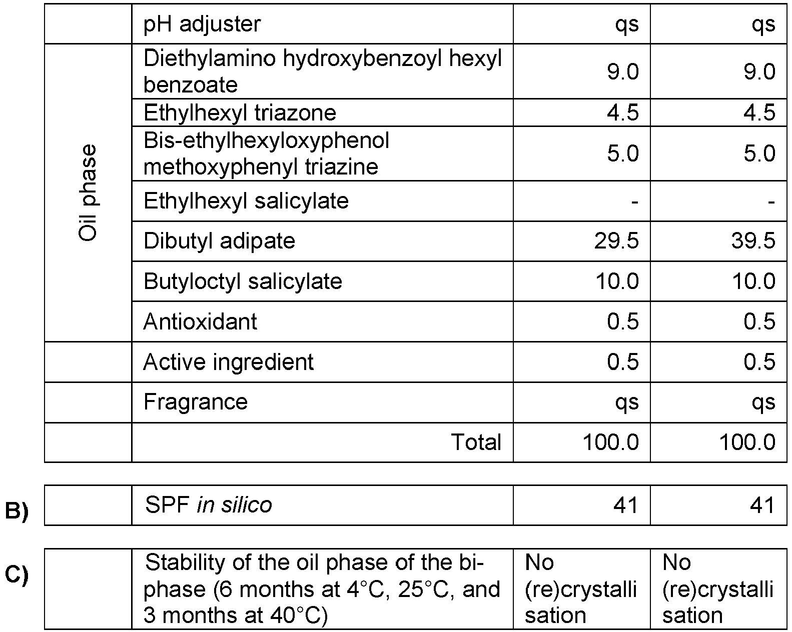

### Manufacturing procedure

- Prepare the water phase by mixing the ingredients together under moderate agitation. Adjust the pH if necessary;
- Prepare the oil phase by mixing the esters and filters together and stir under moderate agitation while heating at 75°C. Cool down to 30°C to add the fragrance and active ingredients under moderate agitation;
- Mix the water phase and the oil phase together.

### Conclusion

From the above examples 1 to 13 we can observe that the filter systems are mainly composed of solid filters, i.e. powder-based with a mass for 100g of composition, ranging between 18.5 and 34.5g (Ex. 7) and that the several emollients show a mass, for 100g of composition, ranging between 41.3 (Ex. 7) and 80g (Ex. 2), i.e. a ratio between the mass of emollients and the mass of the filter system ranging between 1.2 and 4.3.

The emollients selected and their concentrations relative to the UV filter system have shown that a good solubilisation could be achieved, despite the high SPF and the powder based nature of the UV filter system. The above examples are the results of extensive research activities, bearing in mind that achieving a high SPF with filters selected from a limited list of preferred filters, good solubilisation, stability over time and at different temperatures, and good properties among hardness, spreadability, shine and stickiness, as detailed here above, is not trivial.

## Claims

1. Sun protection topical composition comprising:
- a UV filter system achieving a Sun Protection Factor of at least 30;
- at least two ester emollients;
**characterized in that** the at least two ester emollients comprise:
- dibutyl adipate with a mass that is at least 89.5% of a total mass of the UV filter system and
- butyloctyl salicylate with a mass that is at least 24% of a total mass of the UV filter system.

2. Sun protection topical composition according to claim 1, wherein the UV filter system is a powder based by at least 60%, preferably at least 70%, in mass.

3. Sun protection topical composition according to one of claims 1 and 2, wherein the UV filter system comprises at least three different UV filters, preferably each one of the different filters shows a mass that is not less than 33% and/or not more than 300% of the mass of each other one of said different filters.

4. Sun protection topical composition according to any one of claims 1 to 3, wherein the UV filter system comprises filters selected from the group consisting of ethylhexyl triazone, diethylamino hydroxybenzoyl hexyl benzoate, bis-ethylhexyloxyphenol methoxyphenyl triazine, ethylhexyl salicylate, diethylhexyl butamido triazone and homosalate.

5. Sun protection topical composition according to any one of claims 1 to 4, wherein the UV filter system comprises at least one UVA filter and at least one UVB filter, and/or at least one UVA+UVB filter.

6. Sun protection topical composition according to any one of claims 1 to 5, wherein the at least two ester emollients present a total mass of ester emollients comprised between 1.2 and 4.3 times a total mass of the UV filter system.

7. Sun protection topical composition according to any one of claims 1 to 6, wherein the at least two ester emollients further comprise at least one compound selected from the group consisting of triheptanoin, with a mass that is at least 51% of the total mass of the UV filter system, hexyl laurate, with a mass that is at least 32% of the total mass of the UV filter system, isoamyl laurate, with a mass that is at least 65% of the total mass of the UV filter system, and diisopropyl sebacate, with a mass that is at least 40.5%, preferably at least 49%, of the total mass of the UV filter system, or mixtures thereof.

8. Sun protection topical composition according to any one of claims 1 to 7, further comprising at least one oil thickener; so that said sun protection topical composition is solid at room temperature for forming a stick, preferably comprising amino acid based oil gelling agents.

9. Sun protection topical composition according to claim 8, wherein the at least one oil thickener presents a total mass concentration that is comprised between 15% and 25% based on the total sun screen composition, when the UV filter system is of from 23.6 to 40% based on the total sun screen composition.

10. Sun protection topical composition according to claim 8 or 9, further comprising at least one film former, preferably presenting a total mass concentration comprised between 0.5% and 3.5% based on the total sun screen composition.

11. Sun protection topical composition according to claim 8, wherein the UV filter system presents a total mass concentration comprised between 17 and 35% based on the total sun screen composition.

12. Sun protection topical composition according to claim 8, wherein the at least two ester emollients shows a total mass concentration that is comprised between 23.6 and 80%, preferably between 41.3 and 80%, or preferably between 23.6 to 75% based on the total sun screen composition.

13. Sun protection topical composition according to any one of claims 1 to 12, wherein said composition is anhydrous.

14. Sun protection topical composition according to any one of claims 1 to 7, further comprising water, said sun protection topical composition being an emulsion, a gel-cream, a foam or a bi-phasic.

15. Sun protection topical composition according to any one of claims 1 to 14, wherein said composition is free of at least one compound selected from the group consisting of siloxanes, elastomer, octocrylene, butyl methoxydibenzoylmethane, ethylhexyl methoxycinnamate, titanium dioxide and zinc oxide, or mixture thereof.

## Patentansprüche

1. Topische Sonnenschutzzusammensetzung, umfassend:
- ein UV-Filtersystem, das einen Sonnenschutzfaktor von mindestens 30 erreicht;
- mindestens zwei Ester-Emolliente;
**dadurch gekennzeichnet, dass** die mindestens zwei Ester-Emolliente Folgendes umfassen:
- Dibutyladipat mit einer Masse, die mindestens 89,5% der Gesamtmasse des UV-Filtersystems beträgt, und
- Butyloctylsalicylat mit einer Masse, die mindestens 24% der Gesamtmasse des UV-Filtersystems ausmacht.

2. Topische Sonnenschutzzusammensetzung nach Anspruch 1, wobei das UV-Filtersystem zu mindestens 60%, vorzugsweise mindestens 70%, der Masse pulverbasiert ist.

3. Topische Sonnenschutzzusammensetzung nach einem der Ansprüche 1 und 2, wobei das UV-Filtersystem mindestens drei verschiedene UV-Filter umfasst, wobei vorzugsweise jeder der verschiedenen Filter eine Masse aufweist, die nicht weniger als 33% und/oder nicht mehr als 300% der Masse jedes anderen der verschiedenen Filter beträgt.

4. Topische Sonnenschutzzusammensetzung nach einem der Ansprüche 1 bis 3, wobei das UV-Filtersystem Filter umfasst, die aus der Gruppe ausgewählt sind, die aus Ethylhexyltriazon, Diethylamino-Hydroxybenzoyl-Hexylbenzoat, Bis-Ethylhexyloxyphenol-Methoxyphenyltriazin, Ethylhexylsalicylat, Diethylhexyl-Butamido-Triazon und Homosalat besteht.

5. Topische Sonnenschutzzusammensetzung nach einem der Ansprüche 1 bis 4, wobei das UV-Filtersystem mindestens einen UVA-Filter und mindestens einen UVB-Filter und/oder mindestens einen UVA+UVB-Filter umfasst.

6. Topische Sonnenschutzzusammensetzung nach einem der Ansprüche 1 bis 5, wobei die mindestens zwei Ester-Emolliente eine Gesamtmasse an Ester- Emollienten aufweisen, die zwischen dem 1,2- und 4,3-fachen der Gesamtmasse des UV-Filtersystems liegt.

7. Topische Sonnenschutzzusammensetzung nach einem der Ansprüche 1 bis 6, **dadurch gekennzeichnet, dass** die mindestens zwei Ester-Emolliente außerdem mindestens eine Verbindung umfassen, die ausgewählt ist aus der Gruppe bestehend aus Triheptanoin, mit einer Masse, die mindestens 51% der Gesamtmasse des UV-Filtersystems beträgt, Hexyllaurat, mit einer Masse, die mindestens 32% der Gesamtmasse des UV-Filtersystems beträgt, Isoamyllaurat, mit einer Masse, die mindestens 65% der Gesamtmasse des UV-Filtersystems beträgt, und Diisopropylsebacat, mit einer Masse, die mindestens 40,5%, vorzugsweise mindestens 49%, der Gesamtmasse des UV-Filtersystems beträgt, oder Mischungen davon.

8. Topische Sonnenschutzzusammensetzung nach einem der Ansprüche 1 bis 7, ferner umfassend mindestens ein Ölverdickungsmittel, so dass die topische Sonnenschutzzusammensetzung bei Raumtemperatur fest ist, um einen Stift zu bilden, vorzugsweise umfassend Ölgeliermittel auf Aminosäurebasis.

9. Topische Sonnenschutzzusammensetzung nach Anspruch 8, wobei das mindestens eine Ölverdickungsmittel eine Gesamtmassenkonzentration aufweist, die zwischen 15% und 25%, bezogen auf die gesamte Sonnenschutzzusammensetzung, liegt, wenn das UV-Filtersystem 23,6 bis 40%, bezogen auf die gesamte Sonnenschutzzusammensetzung, ausmacht.

10. Topische Sonnenschutzzusammensetzung nach Anspruch 8 oder 9, die außerdem mindestens einen Filmbildner enthält, der vorzugsweise eine Gesamtmassekonzentration zwischen 0,5% und 3,5%, bezogen auf die gesamte Sonnenschutzzusammensetzung, aufweist.

11. Topische Sonnenschutzzusammensetzung nach Anspruch 8, wobei das UV-Filtersystem eine Gesamtmassenkonzentration zwischen 17 und 35 %, bezogen auf die gesamte Sonnenschutzzusammensetzung, aufweist.

12. Topische Sonnenschutzzusammensetzung nach Anspruch 8, wobei die mindestens zwei Ester-Emolliente eine Gesamtmassenkonzentration aufweisen, die zwischen 23,6 und 80%, vorzugsweise zwischen 41,3 und 80% oder vorzugsweise zwischen 23,6 und 75%, bezogen auf die gesamte Sonnenschutzzusammensetzung, liegt.

13. Topische Sonnenschutzzusammensetzung nach einem der Ansprüche 1 bis 12, wobei die Zusammensetzung wasserfrei ist.

14. Topische Sonnenschutzzusammensetzung nach einem der Ansprüche 1 bis 7, die außerdem Wasser enthält, wobei die topische Sonnenschutzzusammensetzung eine Emulsion, eine Creme, ein Schaum oder ein Zweiphasenprodukt ist.

15. Topische Sonnenschutzzusammensetzung nach einem der Ansprüche 1 bis 14, wobei die Zusammensetzung frei ist von mindestens einer Verbindung, ausgewählt aus der Gruppe bestehend aus Siloxanen, Elastomer, Octocrylen, Butylmethoxydibenzoylmethan, Ethylhexylmethoxycinnamat, Titandioxid und Zinkoxid oder einer Mischung davon.

## Revendications

1. Composition topique de protection solaire comprenant :
- un système de filtre UV atteignant un facteur de protection solaire d'au moins 30 ;
- au moins deux émollients esters ;
**caractérisée en ce que** lesdits au moins deux émollients esters comprennent :
- de l'adipate de dibutyle dont la masse représente au moins 89,5 % de la masse totale du système de filtre UV et
- du salicylate de butyloctyle dont la masse représente au moins 24 % de la masse totale du système de filtre UV.

2. Composition topique de protection solaire selon la revendication 1, dans laquelle le système de filtre UV est à base de poudre à au moins 60%, de préférence au moins 70% en masse.

3. Composition topique de protection solaire selon l'une des revendications 1 et 2, dans laquelle le système de filtre UV comprend au moins trois filtres UV différents, et de préférence chacun des différents filtres présente une masse qui n'est pas inférieure à 33% et/ou pas supérieure à 300% de la masse de chacun desdits différents filtres.

4. Composition topique de protection solaire selon l'une quelconque des revendications 1 à 3, dans laquelle le système de filtre UV comprend des filtres sélectionnés dans le groupe constitué de l'éthylhexyl triazone, du diéthylamino hydroxybenzoyl hexyl benzoate, du bis-éthylhexyloxyphénol méthoxyphényl triazine, du salicylate d'éthylhexyle, du diéthylhexyl butamido triazone et de l'homosalate.

5. Composition topique de protection solaire selon l'une quelconque des revendications 1 à 4, dans laquelle le système de filtre UV comprend au moins un filtre UVA et au moins un filtre UVB, et/ou au moins un filtre UVA+UVB.

6. Composition topique de protection solaire selon l'une quelconque des revendications 1 à 5, dans laquelle les au moins deux émollients esters présentent une masse totale d'émollients esters qui est comprise entre 1,2 et 4,3 fois la masse totale du système de filtre UV.

7. Composition topique de protection solaire selon l'une quelconque des revendications 1 à 6, dans laquelle les au moins deux émollients esters comprennent en outre au moins un composé sélectionné dans le groupe constitué de la triheptanoïne, dont la masse représente au moins 51% de la masse totale du système de filtre UV, du laurate d'hexyle, dont la masse représente au moins 32% de la masse totale du système de filtre UV, du laurate d'isoamyle, dont la masse représente au moins 65% de la masse totale du système de filtre UV, et du sébacate de diisopropyle, dont la masse représente au moins 40,5%, de préférence au moins 49%, de la masse totale du système de filtre UV, ou des mélanges de ceux-ci.

8. Composition topique de protection solaire selon l'une quelconque des revendications 1 à 7, comprenant en outre au moins un épaississant à base d'huile, de sorte que ladite composition topique de protection solaire soit solide à température ambiante pour former un stick, comprenant de préférence des agents gélifiants d'huile à base d'acides aminés.

9. Composition topique de protection solaire selon la revendication 8, dans laquelle ledit au moins un épaississant à base d'huile présente une concentration massique totale comprise entre 15% et 25% par rapport à la composition solaire totale, lorsque le système de filtre UV est compris entre 23,6 et 40 % par rapport à la composition de protection solaire totale.

10. Composition topique de protection solaire selon la revendication 8 ou 9, comprenant en outre au moins un filmogène, qui présente de préférence une concentration massique totale comprise entre 0,5 % et 3,5 % par rapport à la composition de protection solaire totale.

11. Composition topique de protection solaire selon la revendication 8, dans laquelle le système de filtre UV présente une concentration massique totale comprise entre 17 et 35% par rapport à la composition de protection solaire totale.

12. Composition topique de protection solaire selon la revendication 8, dans laquelle les au moins deux émollients esters présentent une concentration massique totale comprise entre 23,6 et 80%, de préférence entre 41,3 et 80%, ou de préférence entre 23,6 et 75% par rapport à la composition de protection solaire totale.

13. Composition topique de protection solaire selon l'une quelconque des revendications 1 à 12, dans laquelle ladite composition est anhydre.

14. Composition topique de protection solaire selon l'une quelconque des revendications 1 à 7, comprenant en outre de l'eau, ladite composition topique de protection solaire étant une émulsion, un gel-crème, une mousse ou un biphasique.

15. Composition topique de protection solaire selon l'une quelconque des revendications 1 à 14, dans laquelle ladite composition est dépourvue d'au moins un composé sélectionné dans le groupe constitué des siloxanes, de l'élastomère, de l'octocrylène, du butyl méthoxydibenzoylméthane, du méthoxycinnamate d'éthylhexyle, du dioxyde de titane et de l'oxyde de zinc, ou d'un mélange de ceux-ci.
